**Europäisches Patentamt**

**European Patent Office**

**Office européen des brevets**

(19)

(11) Veröffentlichungsnummer: **0 169 978**
**B1**

(12) **EUROPÄISCHE PATENTSCHRIFT**

(45) Veröffentlichungstag der Patentschrift:
25.01.89

(21) Anmeldenummer: **85104602.9**

(22) Anmeldetag: **16.04.85**

(51) Int. Cl.⁴: **A 61 F 2/34**

(54) **Aus einem inneren Pfannenkörper und einer Aussenschale bestehende Endoprothese für eine Hüftgelenkspfanne.**

(30) Priorität: **16.07.84 CH 3445/84**

(43) Veröffentlichungstag der Anmeldung:
**05.02.86 Patentblatt 86/6**

(45) Bekanntmachung des Hinweises auf die Patenterteilung:
**25.01.89 Patentblatt 89/4**

(84) Benannte Vertragsstaaten:
**AT BE DE FR GB IT NL SE**

(56) Entgegenhaltungen:
**EP-A-0 065 482**
**AU-B-473 057**
**FR-A-2 416 004**
**FR-A-2 519 545**
**GB-A-2 060 118**
**GB-A-2 126 096**

**Anatomie und Physiologie der Haustiere von Klaus Loeffler, Ulmer Verlag, Stuttgart**

(73) Patentinhaber: **GEBRÜDER SULZER AKTIENGESELLSCHAFT**, Zürcherstrasse 9, CH-8401 Winterthur (CH)
Patentinhaber: **Protek AG**, Stadtbachstrasse 64, CH-3001 Bern (CH)

(72) Erfinder: **Spotorno, Lorenzo, Dr.- med., Ospedale Riuniti, I-17024 Finale Ligure (IT)**

(74) Vertreter: **Dipl.- Ing. H. Marsch Dipl.- Ing. K. Sparing Dipl.- Phys.Dr. W.H. Röhl Patentanwälte, Rethelstrasse 123, D-4000 Düsseldorf 1 (DE)**

LIBER, STOCKHOLM 1989

## Beschreibung

Die Erfindung betrifft eine aus einem inneren Pfannenkörper und einer Aussenschale bestehende, für eine zementfreie Verankerung geeignete Endoprothese für eine Hüftgelenkspfanne, bei der der Pfannenkörper mit einem konischen Mantel und die Aussenschale mit einem an den Mantel angepassten Hohlraum versehen sind, wobei die Aussenschale und der Pfannenkörper über eine Verriegelungsstruktur ineinander gehalten sind, wobei ferner auf dem Umfang der aussen mindestens annähernd Halbkugelform aufweisenden Aussenschale eine Anzahl in Meridianrichtung verlaufender Schlitze gleichmässig verteilt sind und wobei ferner die Oberfläche der Aussenschale mit einer aus Vorsprüngen bestehenden Struktur versehen ist.

Eine Prothese der vorstehend genannten Art ist bekannt aus de FR-A-2.416.004; für die Verankerung im Beckenknochen wird die Aussenschale dieser Prothese zunächst mit Hilfe eines Werkzeugs elastisch zusammen- und in Richtung ihrer Achse in den Knochen eingepresst. Nach dem Entfernen des Werkzeugs wird der Pfannenkörper eingetrieben, durch den eine Aufweitung der äquatornahen Bereiche erfolgt.

Es sind daher im wesentlichen zwei verschiedene "Mechanismen" wirksam, durch die die Aussenschale gehalten wird: Zum einen findet im Polbereich ein im wesentlichen in Richtung der Schalenachse wirksames Komprimieren des spongiosen Knochengewebes statt, das einen direkten Übergang der Belastungskräfte auf eine "kompakte" Knochenmasse ermöglicht und darüberhinaus ein rasches Einheilen der Prothese bzw. ihrer Aussenschale fördert. Diese Verdichtung des Gewebes im Polbereich kann dabei zusätzlich noch durch eine sogenannte Spongiosa-Plastik gefördert werden, bei der loses Spongiosa-Gewebe als zusätzliche Hinterfüllung mindestens des Polbereichs der Pfannenprothese verwendet wird, um eine hohe Gewebedichte zu erreichen.

Zum anderen wird die Aussenschale nach dem Lösen des Werkzeugs unter einer radial nach aussen wirkenden Spannung gehalten, durch die die äquatornahen Bereiche in den Beckenknochen eingeklemmt werden. Die Verankerungskräfte wirken also im Polbereich im wesentlichen in Richtung der Schalenachse und in Äquatornähe radial nach aussen.

Die Fixierung der Aussenschale im Knochen - vor allem gegen Torsion um die Schalenachse und gegen ein Abkippen, d.h. gegen Rotationen um in der Äquatorebene liegende momentane Drehachsen unterschiedlicher Richtung - soll dabei durch die Oberflächenstruktur der Aussenschale sichergestellt werden, die in das Gewebe eindringende Vorsprünge aufweist.

Versuche haben gezeigt, dass die Vorsprünge der bisherigen Oberflächenstrukturen im Polbereich lokale Ungleichmässigkeiten der Verdichtung des spongiosen Gewebes bewirken, im Bereich niedriger "geographischer Breiten" jedoch schief in den Knochen eindringen, so dass in ihrer Umgebung örtliche Hohlräume entstehen, in denen die Haftung zwischen Knochen und Implantat vermindert ist.

Aufgabe der Erfindung ist es, eine Oberflächenstruktur zu schaffen, deren Vorsprünge beim Eindringen in den Knochen möglichst weitgehend einerseits eine allseitig gleichmässige Verdichtung des von ihnen verdrängten Gewebes und andererseits überall die Voraussetzung für ein gutes Anhaften und Anwachsen des Gewebes bietet.

Diese Aufgabe wird erfindungsgemäss dadurch gelöst, dass die Vorsprünge der Struktur als in der Aussenschale fest verankerte hinterschneidungsfreie Zotten ausgebildet sind, die im Polbereich der Aussenschale in Richtung der Schalenachse und im äquatornahen Bereich radial zur Aussenschale gerichtet sind.

Da die Vorsprünge sowohl im Polbereich als auch im Äquatorbereich mindestens annähernd in Richtung ihrer Achsen in das Gewebe eindringen und sie darüberhinaus als Zotten sich zu ihrem Fuss hin konisch erweitern, verdrängen und komprimieren sie das Gewebe, wie gefordert, mindestens annähernd nach allen Seiten gleichmässig, ohne dass dabei Hohl- oder Toträume entstehen, was insbesondere durch axialsymmetrisch ausgebildete Zotten unterstützt wird.

Es hat sich als zweckmässig erwiesen, die Zotten unterschiedlicher Richtung durch einen zottenfreien Gürtel voneinander zu trennen, in dem die Meridianschlitze enden. Das Zentrum dieses Gürtels liegt dabei vorteilhafterweise bei einer geographischen Breite von etwa 65°; dieser Wert hat sich als Optimum im Verhältnis zwischen tragendem Polbereich und aufgeweitetem Äquatorbereich erwiesen.

Die Montage der Zotten in der Aussenschale kann schliesslich erleichtert werden, wenn die Einstecktiefe der Zotten in die Aussenchale durch einen Anschlag begrenzt ist. Ihre Befestigung erfolgt dabei vorteilhafterweise durch Löten, Schweissen, Nieten oder Kleben. Vorzugsweise bestehen Aussenschale und Zotten aus Metall, beispielsweise aus Titan oder einer Titanlegierung. Für den Pfannenkörper ist in erster Linie Kunststoff, z. B. Polyäthylen, vorgesehen. Selbstverständlich können Aussenschale und/oder Pfannenkörper zusätzlich - beispielsweise mit die Gewebebildung anregenden oder verschleisshemmenden - Beschichtungen versehen sein.

Obwohl die neue Prothese in erster Linie für einen Einsatz bei einer zementfreien Implantation vorgesehen ist, kann sie selbstverständlich auch in ein Zementbett implantiert werden.

Im folgenden wird die Erfindung anhand eines Ausführungsbeispiels im Zusammenhang mit der Zeichnung näher erläutert.

Fig. 1 ist eine Aufsicht auf die Aussenschale in Richtung der Schalenachse;

Fig. 2 gibt den Schnitt II - II von Fig. 1 wieder;

Fig. 3 zeigt einen, der Fig. 2 entsprechenden Schnitt durch einen Pfannenkörper;

Fig. 4 - 6 geben in vergrössertem Maßstab die Einzelheiten A, B und C aus den Fig. 2 und 3 wieder.

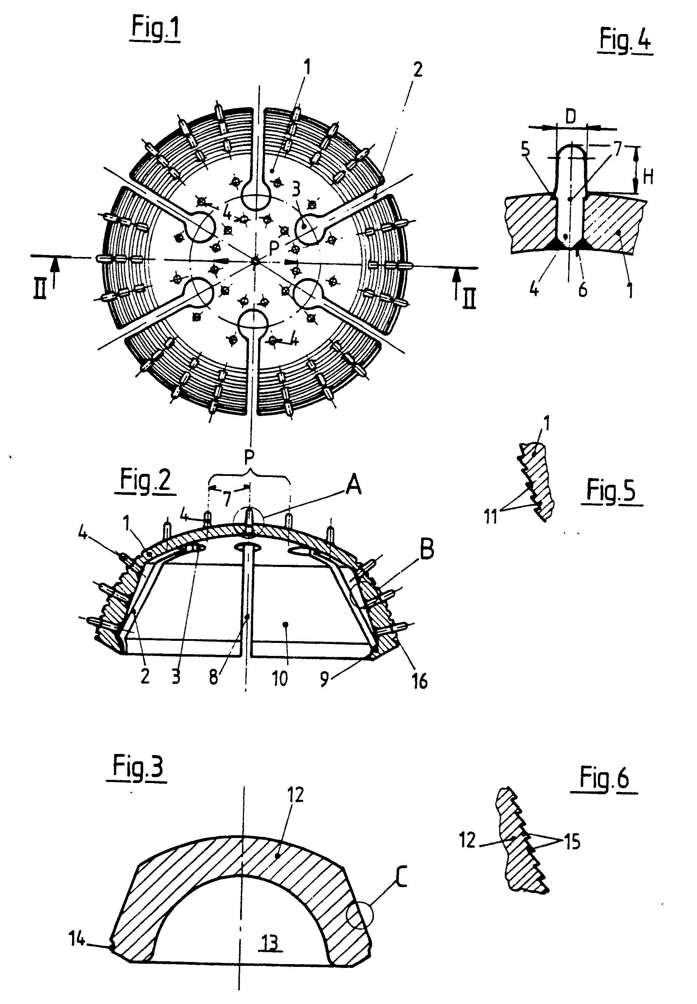

Die Aussenschale 1 (Fig. 1) bildet mindestens annähernd eine Halbkugelschale, in der gleichmässig über den Umfang verteilt Meridianschlitze 2 vorgesehen sind. Diese enden in kreisförmigen Erweiterungen 3, zwischen denen dadurch für elastische Verformungen bevorzugte Bereiche "engeren Querschnittes" entstehen. Die Mittelpunkte der kreisförmigen Erweiterungen 3 liegen etwa auf 65° geographischer Breite.

Aussen ist auf der Aussenschale 1 eine Vielzahl von Vorsprüngen 4 vorgesehen, von denen in Fig. 2 nur einige eingezeichnet sind. Erfindungsgemäss sind die Vorsprünge 4 als Zotten ausgebildet, d.h. ihre Höhe H (Fig. 4) ist mindestens gleich ihrem Durchmesser D und darüberhinaus haben sie eine, sich zum Fuss hin erweiternde konische Form ohne Hinterschneidungen und ohne scharfe Ecken und Kanten. Wie Fig. 4 zeigt besitzen die Zotten 4 an ihrer Wurzel einen Anschlag 5, der unter Umständen in der Aussenschale 1 versenkt sein kann. Durch ihn ist die Einsetztiefe der von aussen in die Aussenschale 1 eingesetzten Zotten 4 bestimmt, die in der Aussenschale 1 in dem gezeigten Beispiel durch Schweisspunkte 6 fixiert sind.

Im Polbereich P der Aussenschale 1 stehen die Achsen 7 der Zotten 4 parallel zur Schalenachse 8, während sie in Gebieten "geringerer Breiten" radial gerichtet sind. Zur Vergrösserung der "Anwachsoberfläche" ist die Aussenchale 1 in Teilbereichen mit in Umfangsrichtung verlaufenden Rillen 16 versehen.

Der Hohlraum 10 der Aussenschale 1 hat im wesentlichen die Form eines Kegelstumpfes, der einseitig durch einen Bogen abgeschlossen ist. Sein seitlicher Mantel ist mit einer Mikroverzahnung 11 (Fig. 5) versehen. Am äquatornahen Rand ist der Hohlraum 10 durch eine über den ganzen Umfang verlaufende Nase 9 abgeschlossen.

Das Gegenstück zum Hohlraum 10 bildet der Pfannenkörper 12, in den die eigentliche Pfannenschale 13 eingearbeitet ist. Er besitzt als Gegenstück zur Nase 9 eine Nut 14, in die die Nase 9 einrastet, wenn der Pfannenkörper 12 in die Aussenschale 1 eingetrieben oder eingepresst ist. Gleichzeitig üben seine ebenfalls mit einer Mikroverzahnung 15 (Fig. 6) versehenen Mantelflächen eine, mindestens teilweise radial gerichtete Aufweitkraft aus, durch die die äquatornahen Bereiche der Aussenschale 1 in den Beckenknochen eingepresst werden. Durch ein Ineinandergreifen der beiden Verzahnungen 11 und 15 sowie das Einrasten der Nase 9 sind die Aussenschale 1 und der Pfannenkörper 12 miteinander verklammert und gegen eine Trennung in Richtung der Achse 8 gesichert.

## Patentansprüche

1. Aus einem inneren Pfannenkörper (12) und einer Aussenschale (1) bestehende, für eine zementfreie Verankerung geeignete Endoprothese für eine Hüftgelenkspfanne, bei der der Pfannenkörper (12) mit einem konischen Mantel und die Aussenschale (1) mit einem an den Mantel angepassten Hohlraum (10) versehen sind, wobei die Aussenschale (1) und der Pfannenkörper (12) über eine Verriegelungsstruktur (9, 14; 11, 15) ineinander gehalten sind, wobei ferner auf dem Umfang der aussen mindestens annähernd Halbkugelform aufweisenden Aussenschale (1) eine Anzahl in Meridianrichtung verlaufender Schlitze (2) gleichmässig verteilt sind und wobei ferner die Oberfläche der Aussenschale (1) mit einer aus Vorsprüngen (4) bestehenden Struktur versehen ist, dadurch gekennzeichnet, dass die Vorsprünge (4) der Struktur als in der Aussenschale (1) fest verankerte, hinterschneidungsfreie Zotten ausgebildet sind, die im Polbereich (P) der Aussenschale (1) in Richtung der Schalenachse (8) und im äquatornahen Bereich radial zur Aussenschale (1) gerichtet sind.

2. Endoprothese nach Anspruch 1, dadurch gekennzeichnet, dass die unterschiedlich gerichteten Zotten (4) durch einen zottenfreien Gürtel im Bereich mittlerer geographischer Breiten der Aussenschale (1) getrennt sind, in dem die Meridian-Schlitze (2) enden.

3. Endoprothese nach Anspruch 2, dadurch gekennzeichnet, dass der zottenfreie Gürtel etwa bei 65° geographischer Breite der Aussenschale liegt.

4. Endoprothese nach einem der Ansprüche 1 bis 3, dadurch gekennzeichnet, dass die Einsetztiefe der Zotten (4) in die Aussenschale (1) durch einen Anschlag (5) begrenzt ist.

## Claims

1. An endoprosthesis for an acetabulum, the endoprosthesis comprising an inner prosthetic socket (12) and an outer shell (1) and being suitable for cement-free fixing, the prosthetic socket (12) having a conical generated surface and the outer shell (1) being formed with a cavity (10) adapted to the conical envelope, the outer shell (1) and the prosthetic socket (12) being retained one in another by way of a locking structure (9, 14; 11, 15), while the periphery of the outer shell (1), the same being in external shape at least approximately hemispherical, is formed with a number of uniformly distributed slots (2) which extend in the meridian direction, the outer shell surface having a structure comprising projections (4), characterised in that the projections (4) of the structure are non-undercut villi rigidly anchored in the outer shell and extend along the shell axis (8) in the pole zone (P) of the outer shell (1) and radially of the outer shell (1) in the zone near the equator.

2. An endoprosthesis according to claim 1, characterised in that the differently directed villi (4) are separated by a villus-free belt in the zone of medium geographic latitudes of the outer shell (1) where the meridian slots (2) terminate.

3. An endoprosthesis according to claim 2, characterised in that the villus-free belt is disposed approximately at 65° of geographical latitude of the outer shell (1).

4. An endoprosthesis according to any of claims 1 - 3, characterised in that the depth of insertion of the villi (4) into the outer shell (1) is limited by an abutment (5).

**Revendications**

1. Endoprothèse pour une cavité glénoïde de la hanche, constituée d'un corps de cavité (12) interne et d'une coque externe (1), convenant à un ancrage sans ciment, dans laquelle le corps de cavité (12) est pourvu d'une enveloppe conique et la coque externe (1) est munie d'une cavité (10) adaptée à l'enveloppe, la coque externe (1) et le corps de cavité (12) étant maintenus l'un dans l'autre par une structure de verrouillage (9, 14; 11, 15), un certain nombre de fentes (2) s'étendant dans la direction méridienne étant en outre uniformément réparties sur le pourtour de la coque externe (1) au moins à peu près semisphérique, et la surface de la coque externe (1) étant pourvue d'une structure constituée de saillies (4), caractérisée en ce que les saillies (4) de la structure sont des villosités sans détalonnage, fermement ancrées dans la coque externe, lesquelles sont dirigées dans la zone polaire (P) de la coque externe vers l'axe (8) de la coque et dans la zone proche de l'équateur, radialement par rapport à la coque externe.

2. Endoprothèse selon la revendication 1, caractérisée en ce que les villosités (4) diversement dirigées sont séparées par une ceinture exempte de villosités dans la zone des latitudes géographiques moyennes de la coque externe (1), dans laquelle aboutissent les fentes méridiennes (2).

3. Endoprothèse selon la revendication 2, caractérisée en ce que la ceinture exempte de villosités se situe à peu près à la latitude géographique de 65° de la coque externe (1).

4. Endoprothèse selon l'une des revendications 1 à 3, caractérisée en ce que la profondeur d'introduction des villosités (4) dans la coque externe (1) est limitée par une butée (5).

Fig.1

Fig.2

Fig.3

Fig.4

Fig.5

Fig.6